(19) Europäisches Patentamt
European Patent Office
Office européen des brevets

(11) EP 1 176 203 A1

(12) **EUROPÄISCHE PATENTANMELDUNG**

(43) Veröffentlichungstag:
**30.01.2002 Patentblatt 2002/05**

(51) Int Cl.[7]: **C12N 15/53**, C12N 9/04, C12N 1/21, C12P 7/02

(21) Anmeldenummer: **01114953.1**

(22) Anmeldetag: **20.06.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **27.07.2000 DE 10037101**

(71) Anmelder: **Degussa Aktiengesellschaft**
**40474 Düsseldorf (DE)**

(72) Erfinder:
- **Riebel, Bettina, Dr.**
  **53181 Leverkusen (DE)**
- **Hummel, Werner, Dr.**
  **52445 Titz (DE)**
- **Bommarius, Andreas, Prof.**
  **30327 Atlanta, GA (US)**

(54) **Enzyme mit verbesserter NAD(H)-Akzeptanz**

(57) Die vorliegende Erfindung richtet sich auf rekombinant hergestelltes Enzym mit gegenüber dem Wildtyp erhöhter NAD(H)-Akzeptanz. Dies wird durch Beibehaltung der basischen Aminosäuren in der Coenzymbindungsstelle des Enzyms und Austausch mindestens einer neutralen Aminosäure (G38D) gegen mindestens eine saure erreicht.
Gensequenzen, Plasmide, Primer, Verfahren und Verwendung.

EP 1 176 203 A1

Printed by Jouve, 75001 PARIS (FR)

## Beschreibung

Die vorliegende Erfindung ist auf ein rekombinant (rec) hergestelltes Enzym gerichtet. Insbesondere betrifft die Erfindung ein solches Enzym, bei dem die NAD(H)-Akzeptanz gegenüber dem Wildtyp erhöht ist. Weiterhin umfaßt die Erfindung die für das rec-Enzym codierenden Gensequenzen, Plasmide und Mikroorganismen, die diese Gensequenzen aufweisen, vorteilhafte Primer, ein Verfahren zur Herstellung der Enzyme und deren Verwendung.

Der Einsatz enzymatischer Verfahren in der Synthese von organischen Verbindungen ist gerade im großtechnischen Maßstab von Vorteil, da sie in Bezug auf die Selektivitäten und Ausbeuten an Produkten den normalen chemischen Verfahren häufig überlegen sind.

U.U. sind derartige enzymatische Verfahren abhängig von sogenannten Co-Faktoren oder Coenzymen. Z.B. sind Alkohol-Dehydrogenasen (ADH) Enzyme, die mit hoher Enantioselektivität Ketone zu den entsprechenden Alkoholen umsetzen. Coenzym solcher Reaktionen ist sehr häufig NADH oder NADPH. Die meisten bekannten ADHs ( z. B. aus Pferdeleber, dem Bakterium Thermoanaerobium brockii) bilden bei Verwendung vergleichbarer Ketone (S)-Alkohole. Aus Lactobacillus-Stämmen sind allerdings zwei (R)-spezifische ADHs bekannt, die sich biochemisch stark ähneln, ein Enzym aus Lactobacillus kefir (EP 91107067.0; DE 4014573) und eins aus L. brevis (EP 0 796 914 A2; DE 196 10 984; DSM 20054).

Eine Einschränkung bei der Verwendung dieser beiden R-spezifischen Enzyme besteht durch die Abhängigkeit vom Coenzym NADP(H). Dieses Coenzym ist beträchtlich instabiler und teurer als das Coenzym NAD(H), zudem existieren keine etablierten und kostengünstigen Regenerierungsverfahren. Wegen der ungewöhnlich breiten Akzeptanz an Ketonen, die von diesen Enzymen praktisch völlig enantiomerenrein umgesetzt werden, sind sie aber für präparative Anwendungen von großem Interesse.

Bei in der Literatur beschriebenen Versuchen, die Coenzym-Spezifität von NADP(H) in Richtung NAD(H) zu verschieben, wurden bislang überwiegend "multiple" Austausche größerer Bereiche vorgenommen, die keine systematische Vorgehensweise erkennen lassen und nicht auf andere NADP(H)-abhängige Enzyme übertragbar sind (Chen, R. et al. (1995) "A highly active decarboxylating dehydrogenase with rationally inverted coenzyme specificity. " Proc. Natl. Acad. Sci. U S A **92**(25): 11666-70; Perham, R. N. et al. (1991). "New enzymes for old: redesigning the coenzyme and substrate specificities of glutathione reductase." Bioessays **13**(10): 515-25; Yaoi, T. et al. (1996). "Conversion of the coenzyme specificity of isocitrate dehydrogenase by module replacement." J. Biochem. (Tokyo) **119**(5): 1014-8). Lediglich eine Publikation (Sem, D. S. and C. B. Kasper (1993) "Interaction with arginine 597 of NADPH-cytochrome P-450 oxidoreductase is a primary source of the uniform binding energy used to discriminate between NADPH and NADH." Biochemistry **32**(43): 11548-58) beschreibt einen singulären Austausch an einer Dehydrogenase (Cytochrom P450-Oxidoreduktase), allerdings wurde dieser in analoger Weise zu WO99/47648 durchgeführt. Die Autoren haben den Austausch einer basischen Aminosäure gegen eine neutrale (Arg597Met) vorgenommen. Die Ergebnisse der Autoren bestätigen eine leichte Verbesserung der NAD-Akzeptanz, doch ist das erhaltene Enzym deutlich instabiler.

Es wurde weiterhin versucht, das Enzym aus L. brevis mit gentechnischen Methoden derart zu verändern, daß es neben NADP(H) auch NAD(H) akzeptiert (WO99/47648). Um diese Änderung in der Coenzym-Akzeptanz zu erreichen, wurden dort im wesentlichen basische Aminosäuren in der Coenzym-Bindungsstelle gegen neutrale ausgetauscht. Dieser Austausch wurde durch Änderung der Nucleotid-Sequenz erreicht, die die (R)-ADH aus L. brevis kodiert. So wurden im Bereich der Coenzym-Bindungsstelle in verschiedenen Kombinationen die basischen Aminosäuren Arginin-38, Lysin-45 und Lysin-48 gegen neutrale (z.B. Methionin, Leucin, Isoleucin, Glycin) ausgetauscht (Dabei handelt es sich um AS-Positionen inklusive des Startcodons ATG). Solche Mutanten des Enzyms akzeptieren zwar tatsächlich auch NAD(H), erwiesen sich aber für eine Anwendung als wenig tauglich, da die Enzymausbeuten relativ gering und vor allem die Stabilitäten dieser neuartigen Enzyme im Vergleich zum NADP(H)-abhängigen Wildtyp-Enzym stark herabgesetzt waren. Auch eine weitere Mutante, bei der zusätzlich zu den oben erwähnten Austauschen basischer Aminosäuren gegen neutrale (Austausche R39L, K48M sowie der Ladungs-neutrale Austausch A9G) auch ein zusätzlicher Austausch einer neutralen gegen eine saure Aminosäure (G38D) vorgenommen wurde, zeigte zwar eine Erweiterung der Coenzym-Akzeptanz in Richtung NAD(H), war aber ebenfalls beträchtlich instabil und nur mit geringen Ausbeuten zu gewinnen.

Aufgabe der vorliegenden Anmeldung war deshalb die Angabe eines allgemeinen Verfahrens und der mithilfe dieses Verfahrens gewonnenen Enzyme, welches es gestattet, bei einer zumindest nicht wesentlichen Verschlechterung der Stabilität der Enzyme, deren eigentlich unnatürliche NAD(H)-Akzeptanz zu vergrößern.

Diese Aufgabe wird durch Angabe der rec-Enzyme mit den kennzeichnenden Merkmalen des Anspruchs 1 gelöst. Ansprüche 2 bis 4 stellen bevorzugte rec-Enzyme unter Schutz. Ansprüche 5 bis 8 richten sich auf die für diese rec-Enzyme codierenden Gensequenzen, Plasmide und Mikroorganismen aufweisend diese Gensequenzen sowie bevorzugte Primer. Ansprüche 9 bis 11 stellen das erfindungsgemäße Verfahren vor, wohingegen Ansprüche 12 und 13 bevorzugte Verwendungen der rec-Enzyme kennzeichnen.

Dadurch, daß man unter Beibehaltung der basischen Aminosäuren in der Coenzymbindungsstelle des Enzyms mindestens eine neutrale Aminosäure gegen mindestens eine saure austauscht, erhält man ein rekombinant hergestelltes Enzym mit gegenüber dem Wildtyp erhöhter NAD(H)-Akzeptanz.
Die vormals vorhandenen natürliche Präferenz für das instabile Coenzym NADP(H) kann also durch den Austausch nur einer Aminosäure in Richtung der bevorzugten und vorteilhaften NAD(H)-Akzeptanz verschoben werden. Dies ist so aus dem Stand der Technik nicht zu entnehmen und mithin sehr überraschend. Im Experiment hat sich gezeigt, daß die Akzeptanz für NAD(H) gegenüber NADP(H) in der erfindungsgemäßen Mutante durch diesen Austausch ca. um den Faktor 300 gesteigert werden kann, ohne die Stabilität des rec-Enzyms zu verschlechtern. Im Gegenteil wird die Temperaturstabilität noch erhöht.

Mithilfe des erfindungsgemäßen Verfahrens können im Prinzip alle dem Fachmann bekannten NADP(H)-abhängigen Enzyme entsprechend getuned werden. Bevorzugt handelt es sich dabei um eine Dehydrogenase, vorzugsweise eine Alkoholdehydrogenase. Weiterhin vorzugsweise erfolgt dies jedoch bei der (R)-ADH aus L. brevis oder L. kefir. Man erhält so vorteilhafte rec-(R)-ADHs aus besagten Organismen mit den oben angesprochenen Vorteilen. Ganz besonders bevorzugt sind solche rec-(R)-ADHs aus L. brevis oder L. kefir, bei denen an Position 38 ein G gegen ein D als Aminosäure ausgetauscht ist. Bei der Positionsangabe ist die Startaminosäure entsprechend dem Codon ATG mitgezählt.

In einer weiteren Ausgestaltung der Erfindung beschäftigt sich diese mit Gensequenzen, welche für eine erfindungsgemäße rec-Enzym codieren.
Gegenstand der Erfindung sind weiterhin Plasmide und Mikroorganismen, welche die erfindungsgemäßen Gensequenzen aufweisen.
Der Mikroorganismus, in den die Gensequenz kloniert wird, dient zur Vermehrung und Gewinnung einer ausreichenden Menge des rekombinanten Enzyms. Die Verfahren hierfür sind dem Fachmann wohlbekannt (Sambrook et al. 1989, Molecular cloning: A Laboratory Manual, 2nd Edition, Cold Spring Harbor Laboratory Press, Balbas P & Bolivar F. 1990, Design and construction of expression plasmd vectors in E.coli, Methods Enzymology 185, 14-37). Als Mikroorganismen können im Prinzip alle dem Fachmann für diesen Zweck in Frage kommende Organismen herangezogen werden. Vorzugsweise sind E. coli-Stämme für diesen Zweck zu benutzen. Ganz besonders bevorzugt sind: E. coli NM 522, JM105, RR1, DH5$\alpha$, TOP 10$^-$ oder HB101. Plasmide, mit denen das die erfindungsgemäße Gensequenz aufweisende Genkonstrukt vorzugsweise in den Wirtsorganismus kloniert wird, sind: pKK-177-3H (Roche Bio-chemicals), pBTac (Roche Biochemicals), pKK-233 (Stratagene) oder pET (Novagen). Weitere sind dem Fachmann im Prinzip ebenfalls bekannt (s.o.a. Literatur, ansonsten immer die entsprechenden molekularbiologischen Fachkataloge).

Die für die PCR notwendigen Primerstränge bilden einen weiteren Teil der vorliegenden Erfindung. Mitumfaßt sind die Sense- und Antisense-Primer codierend für die Aminosäuresequenz TDRHSDVG.

Ein nächster Aspekt der Erfindung betrifft ein Verfahren zur Herstellung von rekombinant hergestellten Enzymen mit gegenüber dem Wildtyp erhöhter NAD(H)-Akzeptanz. Dies wird dadurch erreicht, daß man unter Beibehaltung der basischen Aminosäuren in der Coenzymbindungsstelle des Enzyms mindestens eine neutrale Aminosäure gegen mindestens eine saure austauscht.
Ganz allgemein setzt das erfindungsgemäße Verfahren zur Abwandlung der Enzyme die Kenntnis bzw. einleitende Bestimmung der zu verändernden Aminosäuresequenz des zu verbessernden Enzyms voraus, um einen gezielten Austausch der entsprechenden Aminosäuren vornehmen zu können. Der für die NAD(H)-Spezifitätsverbesserung nützliche Austausch ergibt sich aber auch dann - ohne Vorabkenntnisse der Coenzymbindungsstelle - nach dem trial-and-error-Prinzip, wobei derzeit kommerziell zur Verfügung stehende Fertig-Kits die wesentlichen Teilschritte der gentechnologischen Arbeiten ohne allzu großen Aufwand möglich machen (s. Fachkataloge von Qiagen oder Clontech).
Vorzugsweise wird das erfindungsgemäße Verfahren auf eine Dehydrogenase, vorzugsweise eine Alkoholdehydrogenase, besonders bevorzugt auf die rec-(R)-ADH aus L. brevis oder L. kefir angewandt. Man erhält so vorteilhafte rekombinante Mutanten(rec-Mutanten) der (R)-ADHs (Muteine) aus besagten Organismen. Ganz besonders bevorzugt sind solche rec-(R)-ADHs aus L. brevis oder L. kefir, bei denen an Position 38 ein G gegen ein D als Aminosäure ausgetauscht wird. Bei der Positionsangabe ist die Startaminosäure entsprechend dem Codon ATG mitgezählt.

Ein weiterer Aspekt der Erfindung beschäftigt sich mit der Verwendung eines erfindungsgemäßen rec-Enzyms in einem Verfahren zur Herstellung von enantiomer angereicherten organischen Verbindungen, vorzugsweise enantiomer angereicherte Alkohole.
Vorzugsweise wird die rec-(R)-ADH aus L. brevis oder L. kefir in einem Verfahren zur enantioselektiven Reduktion von Ketonen oder zur enantioselektiven Oxidation von Alkoholen eingesetzt.

Die Herstellung der erfindungsgemäßen rec-Enzyme erfolgt nach dem Fachmann bekannten gentechnologischen Verfahren (z.B. Sambrook et al. 1989 s.o.; Vectors: A Survey of Molecular Cloning Vectors and Their Uses. R.L. Rodriguez & D.T. Denhardt, Eds: 205-225). Bezüglich der allgemeinen Vorgehensweise (PCR und Fusions-PCR, Klonierung, Expression) sei auf die WO99/47684 und das dort zitierte verwiesen.
Der Nachweis der positiven Veränderung der mutierten rec-Enzyme kann durch die Bestimmung der kinetischen Parameter für die Coenzyme $NAD^+$, $NADP^+$, NADH und NADPH über die entsprechenden kinetischen Parameter für das Keton-Substrat erfolgen.

Am Beispiel der rec-(R)-ADH aus L. brevis wird im folgenden die Erfindung (Beispielteil) und deren Vorteile dargestellt.

Vergleicht man biochemisch diejenigen Mutanten, die nach der Anmeldung WO99/47684 erzeugt wurden mit der hier beschriebenen erfindungsgemäßen rec-(R)-ADH, zeigen sich bei der Mutante G38D folgende vorteilhafte Verbesserungen:

Die Mutante zeigt eine beträchtlich verbesserte Temperaturstabilität, dieses Enzym ist sogar deutlich stabiler als das nicht-mutierte NADP(H)-umsetzende Wildtyp-Enzym;

Der Km-Wert ist höher und damit die Affinität schlechter geworden für NADP gegenüber dem Wildtypenzym, dagegen ist der Km-Wert für NAD niedriger und damit die Affinität verbessert worden.

Die Stabilität des Plasmids, das das Gen für die Mutante G38D enthält, ist deutlich besser als die der Plasmide mit den nach WO99/47684 erzeugten Genen;

Die Ausbeute bei der Expression des Gens, das den Austausch für G38D (+ ATG Startcodon) enthält, ist beträchtlich höher als die der nach WO99/47684 erzeugten Enzyme.

Diese durch einen einzigen Austausch erzeugten neuen Eigenschaften der erfindungsgemäßen rec-(R)-ADH ergeben ein Enzym, das für präparative Anwendungen gut geeignet ist. Es akzeptiert das kostengünstigere und stabilere NAD(H) an Stelle von NADP(H), weist selbst eine hohe Stabilität auf und zeigt vorteilhafte biochemische Eigenschaften. Es kann eingesetzt werden sowohl für Reduktionen von Ketonen zu chiralen Alkoholen (Gleichung (1)) als auch für Oxidationsreaktionen (Gl. (2)). Neben Ketonen werden auch Ketoester (z.B. α-, β-, γ-Ketoester) sehr gut genommen.

(1)

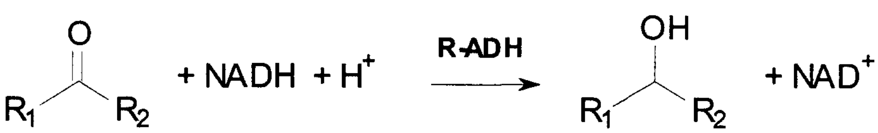


(R)-Alkohol

(2)

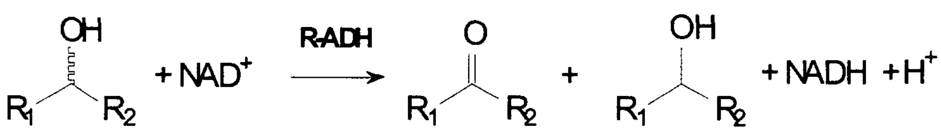


(R,S)-Alkohol (S)-Alkohol

Für präparative Anwendungen nach Gleichung (1) ist die Möglichkeit der Verwendung von NADH besonders vorteilhaft, da für die erforderliche Regenerierung von NADH eine etablierte Standardmethode (Formiat/Formiat-Dehydrogenase) existiert. Da die Bindungsstelle für Ketone bzw. Alkohole durch die Mutation nicht verändert worden ist, kann die bekannte große Anwendungsbreite der rec-(R)-ADH unter Verwendung von NAD(H) in vollem Umfang ausgenutzt werden.

Gensequenzen, welche für Aminosäuresequenzen codieren, umfassen alle Sequenzen, die nach Maßgabe der Degeneration des genetischen Codes möglich erscheinen.

Enantiomer angereichert bezeichnet im Rahmen der Erfindung die Tatsache, daß eine optische Antipode im

Gemisch beider zu >50% vorhanden ist.

SEQUENZPROTOKOLL

<110> Degussa-Hüls AG

<120> Rekombinant hergestelltes Enzym mit verbesserter NAD(H)-Akzeptanz

<130> 000277 AM

<140>
<141>

<160> 3

<170> PatentIn Ver. 2.1

<210> 1
<211> 759
<212> DNA
<213> Künstliche Sequenz

<220>
<223> Beschreibung der künstlichen Sequenz: rec-(R)-Alkoholdehydrogenase

<220>
<221> CDS
<222> (1)..(759)

<400> 1

```
atg tct aac cgt ttg gat ggt aag gta gca atc att aca ggt ggt acg    48
Met Ser Asn Arg Leu Asp Gly Lys Val Ala Ile Ile Thr Gly Gly Thr
  1               5                  10                  15

ttg ggt atc ggt tta gct atc gcc acg aag ttc gtt gaa gaa ggg gct    96
Leu Gly Ile Gly Leu Ala Ile Ala Thr Lys Phe Val Glu Glu Gly Ala
             20                  25                  30

aag gtc atg att acc gac cgg cac agc gat gtt ggt gaa aaa gca gct   144
Lys Val Met Ile Thr Asp Arg His Ser Asp Val Gly Glu Lys Ala Ala
         35                  40                  45

aag agt gtc ggc act cct gat cag att caa ttt ttc caa cat gat tct   192
Lys Ser Val Gly Thr Pro Asp Gln Ile Gln Phe Phe Gln His Asp Ser
     50                  55                  60

tcc gat gaa gac ggc tgg acg aaa tta ttc gat gca acg gaa aaa gcc   240
Ser Asp Glu Asp Gly Trp Thr Lys Leu Phe Asp Ala Thr Glu Lys Ala
 65                  70                  75                  80

ttt ggc cca gtt tct aca tta gtt aat aac gct ggg atc gcg gtt aac   288
Phe Gly Pro Val Ser Thr Leu Val Asn Asn Ala Gly Ile Ala Val Asn
                 85                  90                  95

aag agt gtc gaa gaa acc acg act gct gaa tgg cgt aaa tta tta gcc   336
Lys Ser Val Glu Glu Thr Thr Thr Ala Glu Trp Arg Lys Leu Leu Ala
            100                 105                 110
```

```
gtc aac ctt gat ggt gtc ttc ttc ggt acc cga tta ggg att caa cgg     384
Val Asn Leu Asp Gly Val Phe Phe Gly Thr Arg Leu Gly Ile Gln Arg
        115                 120                 125

atg aag aac aaa ggc tta ggg gct tcc atc atc aac atg tct tcg atc     432
Met Lys Asn Lys Gly Leu Gly Ala Ser Ile Ile Asn Met Ser Ser Ile
    130                 135                 140

gaa ggc ttt gtg ggt gat cct agc tta ggg gct tac aac gca tct aaa     480
Glu Gly Phe Val Gly Asp Pro Ser Leu Gly Ala Tyr Asn Ala Ser Lys
145                 150                 155                 160

ggg gcc gta cgg att atg tcc aag tca gct gcc tta gat tgt gcc cta     528
Gly Ala Val Arg Ile Met Ser Lys Ser Ala Ala Leu Asp Cys Ala Leu
                165                 170                 175

aag gac tac gat gtt cgg gta aac act gtt cac cct ggc tac atc aag     576
Lys Asp Tyr Asp Val Arg Val Asn Thr Val His Pro Gly Tyr Ile Lys
            180                 185                 190

aca cca ttg gtt gat gac cta cca ggg gcc gaa gaa gcg atg tca caa     624
Thr Pro Leu Val Asp Asp Leu Pro Gly Ala Glu Glu Ala Met Ser Gln
        195                 200                 205

cgg acc aag acg cca atg ggc cat atc ggt gaa cct aac gat att gcc     672
Arg Thr Lys Thr Pro Met Gly His Ile Gly Glu Pro Asn Asp Ile Ala
    210                 215                 220

tac atc tgt gtt tac ttg gct tct aac gaa tct aaa ttt gca acg ggt     720
Tyr Ile Cys Val Tyr Leu Ala Ser Asn Glu Ser Lys Phe Ala Thr Gly
225                 230                 235                 240

tct gaa ttc gta gtt gac ggt ggc tac act gct caa tag                 759
Ser Glu Phe Val Val Asp Gly Gly Tyr Thr Ala Gln
                245                 250


<210> 2
<211> 252
<212> PRT
<213> Künstliche Sequenz
<223> Beschreibung der künstlichen Sequenz:
      rec-(R)-Alkoholdehydrogenase

<400> 2
Met Ser Asn Arg Leu Asp Gly Lys Val Ala Ile Ile Thr Gly Gly Thr
  1               5                  10                  15
Leu Gly Ile Gly Leu Ala Ile Ala Thr Lys Phe Val Glu Glu Gly Ala
             20                  25                  30
Lys Val Met Ile Thr Asp Arg His Ser Asp Val Gly Glu Lys Ala Ala
         35                  40                  45
Lys Ser Val Gly Thr Pro Asp Gln Ile Gln Phe Phe Gln His Asp Ser
     50                  55                  60
Ser Asp Glu Asp Gly Trp Thr Lys Leu Phe Asp Ala Thr Glu Lys Ala
 65                  70                  75                  80
Phe Gly Pro Val Ser Thr Leu Val Asn Asn Ala Gly Ile Ala Val Asn
                 85                  90                  95
Lys Ser Val Glu Glu Thr Thr Thr Ala Glu Trp Arg Lys Leu Leu Ala
            100                 105                 110
```

```
Val Asn Leu Asp Gly Val Phe Phe Gly Thr Arg Leu Gly Ile Gln Arg
        115                 120                 125
Met Lys Asn Lys Gly Leu Gly Ala Ser Ile Ile Asn Met Ser Ser Ile
    130                 135                 140
Glu Gly Phe Val Gly Asp Pro Ser Leu Gly Ala Tyr Asn Ala Ser Lys
145                 150                 155                 160
Gly Ala Val Arg Ile Met Ser Lys Ser Ala Ala Leu Asp Cys Ala Leu
                165                 170                 175
Lys Asp Tyr Asp Val Arg Val Asn Thr Val His Pro Gly Tyr Ile Lys
            180                 185                 190
Thr Pro Leu Val Asp Asp Leu Pro Gly Ala Glu Glu Ala Met Ser Gln
        195                 200                 205
Arg Thr Lys Thr Pro Met Gly His Ile Gly Glu Pro Asn Asp Ile Ala
    210                 215                 220
Tyr Ile Cys Val Tyr Leu Ala Ser Asn Glu Ser Lys Phe Ala Thr Gly
225                 230                 235                 240
Ser Glu Phe Val Val Asp Gly Gly Tyr Thr Ala Gln
                245                 250


<210> 3
<211> 8
<212> PRT
<213> Künstliche Sequenz

<220>
<223> Beschreibung der künstlichen Sequenz:
      Aminosäuresequenz der Primer

<400> 3
Thr Asp Arg His Ser Asp Val Gly
  1               5
```

Beispiele:

Der Ergebnisteil gliedert sich in folgende Abschnitte:

1) Beschreibung der Herstellung der Mutante recADHG38D

2) Charakterisierung der neuen NAD-Mutante recADHG38D

3) Vergleich der biochemischen Eigenschaften der neuen Mutante mit einer Mutante hergestellt nach WO99/47684 und dem Wildtypenzym.

4) Substratspektrum und Nachweis der Enantioslektivität der Mutante

**Beispiel 1) Beschreibung der Herstellung der Mutante recADHG38D:**

Als Templat diente für die Herstellung dieser Mutante das Gen des Wildtypenzyms, das kloniert in E.coli vorliegt.

Ausgehend von der Primärsequenz des Wildtypenzyms und unter Berücksichtigung der Kenntnis der Raumstruktur dieser Wildtyp-ADH wurden genetische Primer definiert und verwendet, so dass mit der Methode der "Polymerase-Ketten-Reaktion (PCR)" an der Position 38 ein Austausch von Glycin nach Asparaginsäure durchgeführt wurde.

Primer für die gerichtete Mutagenese der Änderung der Cofaktorspezifität von NADP nach NAD (der gewünschte Aminosäure-Austausch ist kursiv hervorgehoben):
5'Primer mit dem Aminosäureaustausch G38Ds:

5´ACC GAC CGG CAC AGC GAT GTT GGT 3´

T *D* R H S D V G

3'Primer mit dem Aminosäureaustausch G38Das:

5´ACC AAC ATC GCT GTG CCG GTC GGT 3´

G V D S H R *D* T

Um eine Mutation erfolgreich durchzuführen, muss der für den Aminosäureaustausch verantwortliche Nucleotidaustausch auf beiden DNA-Strängen erfolgen, sowohl auf dem leading strand (s = sense) als auch auf dem lagging strand (as = antisense). Für die Mutations-PCR bedeutet dies, dass 2 Genstücke generiert werden, einer vom N-Terminus des Gens bis zum Aminosäureaustausch und einer vom Aminosäureaustausch bis zum C-Terminus des Gens. Diese beiden Genstücke haben dann einen überlappenden Bereich im oben genannten Primer mit dem Aminosäureaustausch, d.h. die oben genannten Aminosäuren von TDRHSDVG haben beide Genstücke gemeinsam. Über diesen gemeinsamen Bereich können die beiden Genstücke dann in einer 2., sogenannten Fusions-PCR, fusioniert werden.

PCR mit den neuen mutationsspezifischen Primern zur Herstellung des kurzen und langen Fragments:

| PCR | Template | 5'Primer | 3'Primer | dNTP | Puffer | DNAzyme | $H_2O$ | Temp. |
|---|---|---|---|---|---|---|---|---|
| 1 | recADH WT 2µl | G38Ds 100pmol | Bras 100pmol | 16 µl | 10 µl | 0.5 µl | 69.5 µl | 56°C |
| 2 | recADH WT 2 µl | BRs 100pmol | G38Das 100pmol | 16 µl | 10 µl | 0.5 µl | 69.5 µl | 56°C |

Die aus dieser PCR entstandenen Genstücke wurden in der Fusions-PCR zusammengesetzt. Dazu wurden gleiche pmol-Enden an Templat aus PCR 1 und PCR 2 zusammenpipettiert und ansonsten die gleichen Zutaten eingesetzt wie oben, ausser den Primern.

Die ersten 5 Cyclen der PCR wurden ohne jegliche Primer durchgeführt, nach dem 5. Cyclus wurden je 100 pmol von BRs (N-Terminus des Gens) und BRas (C-Terminus des Gens) hinzugeführt, und weitere 25 Cyclen absolviert. Durch die ersten 5 Cyclen ohne Primer wurde gewährleistet, dass nur fusionierte Genstücke als Templat für die Polymerase dienen können. Die Amplifikation setzte dann nach 5 Cyclen mit Zugabe der genspezifischen Primer ein.

Auf diese Weise können Gene mit Punktmutationen auf beiden DNA-Strängen generiert werden.

| Fusions PCR | Template | 5'Primer | 3'Primer | dNTP | Puffer | DNAzyme | $H_2O$ | Temp |
|---|---|---|---|---|---|---|---|---|
| 3 | PCR 1 1pmol + PCR 2 1pmol | BRs 100pmol | BRAS 100pmol | 16 µl | 10 µl | 0.5 µl | 59.5 µl | 52°C |

Das Fusionsprodukt (= recADH-MuteinG38D) wurde aus dem Gel isoliert (Gel Extraction Kit, Qiagen) und aufgereinigt. Anschliessend wurde das Gen entsprechend seiner angefügten N- und C-terminalen Restriktionsschnittstellen (Eco R1 und HindIII) geschnitten und erneut über Gelelektrophorese isoliert und aufgereinigt. (Für nähere Beschreibung siehe Patent WO99/47684).

Der hier verwendete kommerzielle Vektor pBTAC2 (Roche Diagnostics; früher Boehringer Mannheim, s. Fig.

7) wurde ebenfalls mit EcoR1 und HindIII restringiert, und somit für die Klonierung mit dem Vektor vorbereitet.

Klonierung in den Vektor pBTac2:

Das restringierte Mutein wurde mittels des Rapid Ligation Kits (Fa. Roche Diagnostics) in den Vektor pBTAC2 ligiert und anschliessend in kompetente E.coli JM105 Zellen transformiert (60 sec 42°C Heatshock) (wahlweise auch in E. coli SG13009 Zellen (Qiagen), die zusätzliche Repressorplasmide mit Neomycinresistenz enthalten, Plasmid pREP4, kommerziell erhältlch von Qiagen).

Die erfolgreich transformierten Klone wurden auf ihre Expressionsleistung hin getestet.

Expression des Muteins G38D:

Das Mutein wurde mit 1 mM IPTG bei OD 0.5 im Schüttelkolben (LB-Medium) induziert, und die Zellen nach 24 h Expression geerntet. Als Selektionsdruck fungiert Ampicillin.

Das Mutein G38D wurde mit sehr guter Expressionsleistung gebildet, sie war vergleichbar mit der Expression des Wildtyp-Enzyms. Im Rohextrakt der rekombinanten Zellen wurde ca. 30-40 % des gesamten Proteins als rekombinantes ADH-Mutein G38D gebildet, die Volumenaktivität (getestet mit Acetophenon/NADH) beträgt 23 U/ml.

**Beispiel 2) Reinigung und biochemische Charakterisierung der Mutante recADHG38D:**

Das Mutein wurde bis zum nahezu homogenen Protein aufgereinigt und charakterisiert.

**Reinigung der recADH-MuteinG38D:**

Der das Mutein enthaltende E. coli-Stamm wurde mit 0.1 M Na-acetat pH 4.5 aufgeschlossen (Glasperlenaufschluss, IMA Disintegrator S, 4000 rpm, 20 min, 4°C) und der Zellbrei anschliessend bei 13000 rpm zentrifugiert (Sorvall SS34 Rotor, 4°C, 10 min). Der zellfreie Überstand enthält das Enzym (Rohextrakt). Dieser Rohextrakt wurde mit $(NH_4)_2SO_4$ auf 0.6 M eingestellt und auf eine mit 50 mM TEA pH 7.0 + 0.6 M Ammoniumsulfat + 1 mM $MgCl_2$ equilibrierte Phenylsepharosesäule (25 ml SV, Pharmacia) aufgetragen. Das Protein wurde mit einem fallenden Salzgradienten bis 0 M Ammoniumsulfat eluiert. Die aktiven Fraktionen wurden vereinigt, und über Ultrafiltration (Amicon Rührzelle) eingeengt. Dieser aktive Pool wurde mit Ammoniumsulfat bis 1.2 M versetzt, und auf eine gegen 50 mM TEA pH 7.0 + 1 mM $MgCl_2$ + 1.2 M Ammoniumsulfat equilibrierte Octylsepharosesäule aufgegeben. Das Protein wurde erneut mit einem fallenden Gradienten bis 0 M Ammoniumsulfat eluiert. Das aktive Eluat dieser Säule wurde für die Charakterisierungen verwendet.

Reinigungstabelle:

| Probe | Aktivität[U/ml] | Protein[mg/ml] | spez. Akt.[U/mg] | Ausbeute [%] | Faktor | Σ U |
|---|---|---|---|---|---|---|
| Rohextrakt | 23.2 | 5.78 | 4.02 | 100 | 1 | 511 |
| Phenyls. | 50 | 17.18 | 2.88 | 19 | 0.71 | 98 |
| Octyls. | 8.36 | 1.22 | 6.85 | 4 | 1.7 | 20 |

SDS-PAGE der MuteinG38D - Reinigung (Fig.1):

| | | |
|---|---|---|
| 1 | RE | 115 µg |
| 2 | PS | 340 µg |
| 3 | Octyll | 31.6 µg |
| 4 | Octyl2 | 48.8 µg |

Wie aus der Abbildung ersichtlich, wird das Mutein G38D der recADH stark überexprimiert, die geringere Volumenaktivität gegenüber dem Wildtyp beruht nicht auf einer schwächeren Expressionsleistung. Spur 4 entspricht

dem gewählten Octylpool in der obigen Reinigungstabelle.

**Charakterisierung der recADH Mutein G38D:**

Das Mutein wurde in bezug auf pH-Optimum, pH-Stabilität, Temperaturoptimum, Temperaturstabilität, Km-Werte für die oxidative Richtung, Kcat, und Kcat/Km charakterisiert.

Diese Kriterien wurden in Vergleich gesetzt zum Wildtypenzym und auch zu dem Mutein 2(Mutante 2; recADH R39L,K49M,A10G (mit zusätzlichem Startcodon gezählt) die in der Anmeldung WO99/47684 erzeugt und beschrieben ist.

pH-Optimum des Muteins G38D (Fig. 2) :

Das pH-Optimum des Muteins, das pH-Optimum der Reduktionsrichtung liegt bei 5.5, das der Oxidationsrichtung bei 6.5

pH-Stabilität des Muteins G38D (Fig. 3):

Die pH-Stabilität wurde je nach pH-Bereich in unterschiedlichen Puffern durchgeführt, das Enzym ist stabil für mindestens 24 h zwischen 6.5 und 8.5, dabei ist deutlich zu sehen, dass sich TEA Puffer nicht für die Lagerstabilität eignet. Der Puffer zeigt bei gleichen pH-Werten immer niedrigere Werte an als die anderen.

Temperaturstabilität (Fig. 4):

Die Temperaturstabilität wurde mit Proben, die +/- 50 % Glycerin (Endkonzentration) enthielten, durchgeführt. 50 µl Enzymprobe wurde mit ausreichend Paraffinöl überschichtet, um ein Verdampfen bei hohen Temperaturen zu verhindern. Glycerin ist für eine längere Stabilität des Enzyms unbedingt notwendig, da es sonst bei Temperaturen um 50°C denaturiert. Im Vergleich wurde das Wildtypenzym mit Glycerin gemessen, die Mutante 2 (R39L K49M A10G; WO99/47684) ohne Glycerinzusatz.

Die Temperaturstabilität wurde bei 42°C gemessen, die Halbwertszeit des Muteins beträgt 257 h mit Glycerinzusatz.

| t, min | A, U/ml | In A | In A calc. | A calc., U/ml |
|---|---|---|---|---|
| 0 | 15 | 2,7080502 | 2,36981758 | 10,695441 |
| 1440 | 7,48 | 2,01223279 | 2,30501677 | 10,0243463 |
| 2880 | 8,12 | 2,09433015 | 2,24021596 | 9,39536008 |
| 4320 | 9,2 | 2,21920348 | 2,17541515 | 8,8058401 |
| 11520 | 6,74 | 1,90805992 | 1,8514111 | 6,36880021 |
| Steigung:= | | -4,5001E-05 | | |
| Achsensabschnitt:= | 2,36981758 | | | |

zugehörige Tabelle zur Fig. 4

Die Temperaturstabilität des Muteins wurde bei 30°C gemessen, die Halbwertszeit beträgt 148h (Fig. 5).

| t, min | A, U/ml | In A | In A calc. | A calc., U/ml |
|---|---|---|---|---|
| 0 | 15 | 2,7080502 | 2,65518596 | 14,2276316 |
| 1440 | 11,32 | 2,42657107 | 2,54298444 | 12,7175693 |
| 2880 | 11,24 | 2,41947884 | 2,43078292 | 11,3677787 |
| 4320 | 11,14 | 2,41054223 | 2,3185814 | 10,1612493 |
| 11520 | 5,7 | 1,74046617 | 1,7575738 | 5,79835233 |

zugehörige Tabelle zu Fig. 5

Temperaturoptimum:

Das Temperaturoptimum wurde im Testansatz in der Küvette bestimmt. Gemessen wurde die Aktivität mit Acetophenon und NADH (Fig. 6). Das Temperaturoptimum des Muteins G38D liegt bei 40°C.

**Beispiel 3) Vergleich der biochemischen Eigenschaften der Mutante recADHG38D mit einer Mutante hergestellt nach WO99/47684 und dem Wildtypenzym**

Die Km-Werte und alle sich auf Km- bzw. Vmax-Werte beziehenden Daten werden in der nachfolgenden Gesamttabelle dargestellt, die Berechnung der Werte erfolgte mittels nichtlinearer Regression mit dem Programm ORIGIN.

Tabelle:

| Zusammenfassung und Vergleich aller Charakteristika der Muteine G38D und Mutante 2 (WO99/47684) mit dem Wildtypenzym | | | |
|---|---|---|---|
| Charakteristika | Wildtypenzym | Mutante 2 | Mutein G38D |
| pH Opt. Redukt. | 6,5 | 6,5 | 5,5 |
| pH Opt. Oxid. | 8,0 | 6,5 | 6,5 |
| pH Stab. 24 h | 4,5-9,0 (70%) | 5,5-8,5 (70%) | 6,5-8,5 (80%) |
| Temp.opt. [°C] | 55 | 50 | 40 |
| Temp.stab.30°C | 150 h * | 16,5 h | 148 h * |
| Temp.stab.42°C | 7,15 h * | 0,19 h | 257 h * |
| Km NAD [mM] | 2,94 | 0,77 | 0,89 |
| Km NADP [mM] | 0,24 | 0,11 | 14,04 |
| Vmax NAD [nMol/ml*s] | 467 | 439 | 236 |
| Vmax NADP [nMol/ml*s] | 1420 | 623 | 402 |
| kcat NAD [$s^{-1}$] | 21,4 | 33,11 | 34,57 |
| kcat NADP [$s^{-1}$] | 65,2 | 46,98 | 58,88 |
| kcat/Km NAD [$s^{-1}$*$mM^{-1}$] | 7,3 | 43 | 38,84 |
| kcat/Km NADP [$s^{-1}$*$mM^{-1}$] | 270 | 427 | 4 |
| NAD:NADP*** | 0,03:1 | 0,1:1 | 10:1 |

* mit 50 % Glycerin

*** Berechnet wurde das Verhältnis von kcat/Km für NAD zu kcat/Km für NADP als quantitatives Maß für die Akzeptanz der beiden Coenzyme.

Die Verbesserungen des Muteins G38D liegt in einer deutlich verbesserten Akzeptanz von NAD. Die zusammenfassende Tabelle stellt klar, daß das Wildtyp-Enzym NAD nur in einem Verhältnis von 0,03 : 1 umsetzen kann, während das vorab beschriebene neue Mutein NAD 10-fach besser nimmt als NADP. Darüberhinaus hat das erfindungsgemäße Mutein eine deutlich verbesserte Temperaturstabilität gegenüber dem Wildtypenzym (beide mit Glycerin im Puffer gemessen), insbesondere bei höheren Temperaturen (42°C). Die Temperaturstabilitäten sind immer als Halbwertszeiten dargestellt, wobei t1/2 die Zeit bestimmt, wo noch 50 % Restaktivität zu messen sind. Eine gute Temperaturstabilität wird allgemein als Maß für eine gute Langzeitstabilität unter Produktionsbedingungen angesehen.

**Beispiel 4) Substratspektrum der Mutante recADHG38D**

Es ist bekannt, daß das NADP(H)-abhängige Wildtyp-Enzym eine Vielzahl von Ketonen, Ketoestern und anderen Carbonylgruppen-enthaltenden Verbindungen stereospezifisch reduzieren kann. Im folgenden werden nur einige wenige ausgewählte Ketoverbindungen als Substrate getestet, um zu bestätigen, daß sich die Substraterkennungsregion durch die Veränderung der Coenzym-Bindungsstelle nicht prinzipiell geändert hat. Dazu werden die Ketoverbindungen in folgendem Ansatz getestet (1 ml Gesamtvolumen):
10 mM Keto-Substrat; 1 mM $MgCl_2$ x 6 $H_2O$; 0,4 mM NADH; 960 µl Triethanolamin-Puffer, 50 mM, pH 7,0; 10 µl Enzym

(Mutein G38D) ; partiell gereinigt (Phenylsepharose; s.o.).

Die Aktivität wird photometrisch bei 340 nm (30°C) bestimmt. Die folgende Tabelle faßt die Aktivitätswerte zusammen.

Tab.:

| Substratspektrum der NAD-Mutante G38D (Aktivitäten relativ auf Acetophenon (=21,08 U/ml) bezogen) | |
|---|---|
| Substrat | Aktivität, relativ [%] |
| Acetophenon | 100 |
| 4-Cl-Acetophenon | 68 |
| 2-Hexanon | 169 |
| 2-Heptanon | 207 |
| 2-Methylcyclohexanon | 334 |
| Acetessigsäure-methylester | 188 |
| Acetessigsäure-ethylester | 88 |
| 4-Chlor-acetessigsäureethylester | 228 |
| Brenztraubensäure-methylester | 191 |
| Brenztraubensäure-ethylester | 260 |
| 2-Oxobuttersäureethylester | 137 |
| 3-Methyl-2-oxobuttersäure-ethylester | 84 |
| Benzylpyruvatethylester | 13 |
| Phenylglyoxylsäuremethylester | 10 |
| 3-Oxovaleriansäuremethylester | 127 |

**Beispiel 5) Nachweis der Stereoselektivität der recADH Mutein G38D:**

Für einzelne, ausgewählte Keto-Substrate soll die Enantiomerenreinheit des durch Reduktion gebildeten Produkts nachgewiesen werden. Dazu werden die Substrate unter Coenzym-Regenerierung weitgehend vollständig umgesetzt und die Enantiomerenreinheit des Produkts mittels Gas-Chromatografie bestimmt.

Umsetzung (1 ml gesamt):

10 mM Keto-Substrat; 1 mM $MgCl_2$ x 6 $H_2O$; 1 mM NADH; 100 mM Na-formiat; 0,8 U Formiat-Dehydrogenase; 2 U NAD-Mutante (Units mit Acetophenon/NADH photometrisch bestimmt); 680 µl Triethanolamin-Puffer, 50 mM, pH 7,0.

Nach 30 und 120 min werden jeweils Proben entnommen (50 µl), mit 100 µl Ethylacetat zum Extrahieren des Produkts versetzt und die Ethylacetat-Phase (1 µl) für die GC-Analytik verwendet. Die Enantiomerentrennung durch GC wird für jedes Produkt durch Applikation des Racemats überprüft. Die Reinheit des Produkt wird als ee-Wert angegeben nach:

$$ee(R) = [R] - [S] / [R] + [S]$$

Ist kein S-Enantiomer nachweisbar, wird der ee-Wert als >99% angegeben.

GC-Analytik:

Säule: CP-Chirasil-DEX CB Länge: 25 m, Durchmesser: 25 um (Fa. Chrompack). Temperaturprogramm: 5 min bei 60°C, dann 5°C/min bis auf 190°C (Für Hexanon/Hexanol: 30 min bei 60°C, dann 10°C/min auf 195°C). Säulenfluß 1,3 ml/min; Gas: Helium

Die folgende Tabelle faßt die Daten zur Produktreinheit zusammen.

Tab.:

| Nachweis der Enantiomerenreinheit der durch enzymatische Reduktion gebildeten Produkte | | |
|---|---|---|
| Substrat (Retentionszeit) | Retentionszeit des Produkts | ee-Wert [%] des Produkts |
| Acetophenon (16,92 min) | 20,82 min | >99% |
| 4-Cl-Acetophenon (21,84 min) | 25,74 min | >99% |
| 2-Oxobuttersäureethylester (10,39 min) | 13,91 min | >99% |
| 2-Hexanon | 21,77 min | >99% |
| 2-Heptanon | 14,22 min | >99% |

**Patentansprüche**

**1.** Rekombinant hergestelltes Enzym mit gegenüber dem Wildtyp erhöhter NAD(H)-Akzeptanz,
**dadurch gekennzeichnet, daß**
unter Beibehaltung der basischen Aminosäuren in der Coenzymbindungsstelle des Enzyms mindestens eine neutrale Aminosäure gegen mindestens eine saure ausgetauscht ist.

**2.** rec-Enzym nach Anspruch 1,
**dadurch gekennzeichnet, daß**
es sich um eine Dehydrogenase, vorzugsweise eine Alkoholdehydrogenase handelt.

**3.** rec-Enzym nach Anspruch 2,
**dadurch gekennzeichnet, daß**
es sich um eine rec-(R)-ADH aus L. brevis oder L. kefir handelt.

**4.** rec-Enzym nach Anspruch 3,
**dadurch gekennzeichnet, daß**
der Austausch G38D betrifft.

**5.** Gensequenz codierend für ein rec-Enzym gemäß einem oder mehreren der Ansprüche 1 bis 4.

**6.** Plasmid aufweisend die Gensequenz nach Anspruch 5.

**7.** Mikroorganismus aufweisend die Gensequenz nach Anspruch 5.

**8.** Sense- und Antisense-Primer codierend für: TDRHSDVG

**9.** Verfahren zur Herstellung eines rec-Enzyms gemäß Anspruch 1, 2, 3 und/oder 4,
**dadurch gekennzeichnet, daß**
man unter Beibehaltung der basischen Aminosäuren in der Coenzymbindungsstelle des Enzyms mindestens eine neutrale Aminosäure gegen mindestens eine saure austauscht.

**10.** Verfahren nach Anspruch 9,
**dadurch gekennzeichnet, daß**
es sich um eine Dehydrogenase, vorzugsweise eine Alkoholdehydrogenase, besonders bevorzugt um die rec-(R)-ADH aus L. brevis oder L. kefir handelt.

**11.** Verfahren nach Anspruch 10,
**dadurch gekennzeichnet, daß**
der Austausch G38D betrifft.

**12.** Verwendung eines rec-Enzyms gemäß Anspruch 1, 2, 3 und/oder 4 in einem Verfahren zur Herstellung von enantiomer angereicherten organischen Verbindungen.

**13.** Verwendung des rec-Enzyms gemäß Anspruch 3 und/oder 4 in einem Verfahren zur enantioselektiven Reduktion von Ketonen oder zur enantioselektiven Oxidation von Alkoholen.

Fig. 1:

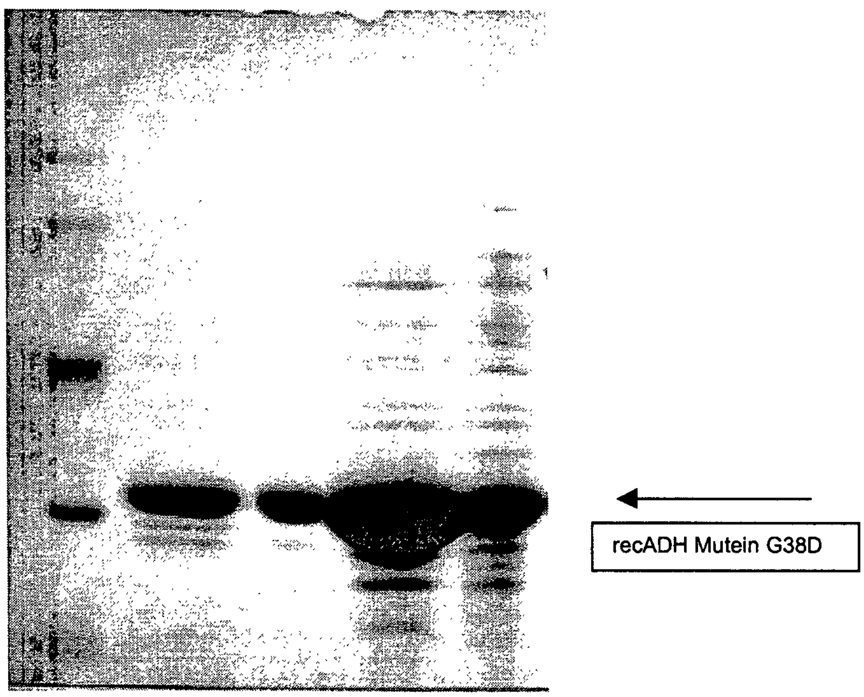


5 4 3 2 1

Fig. 2:

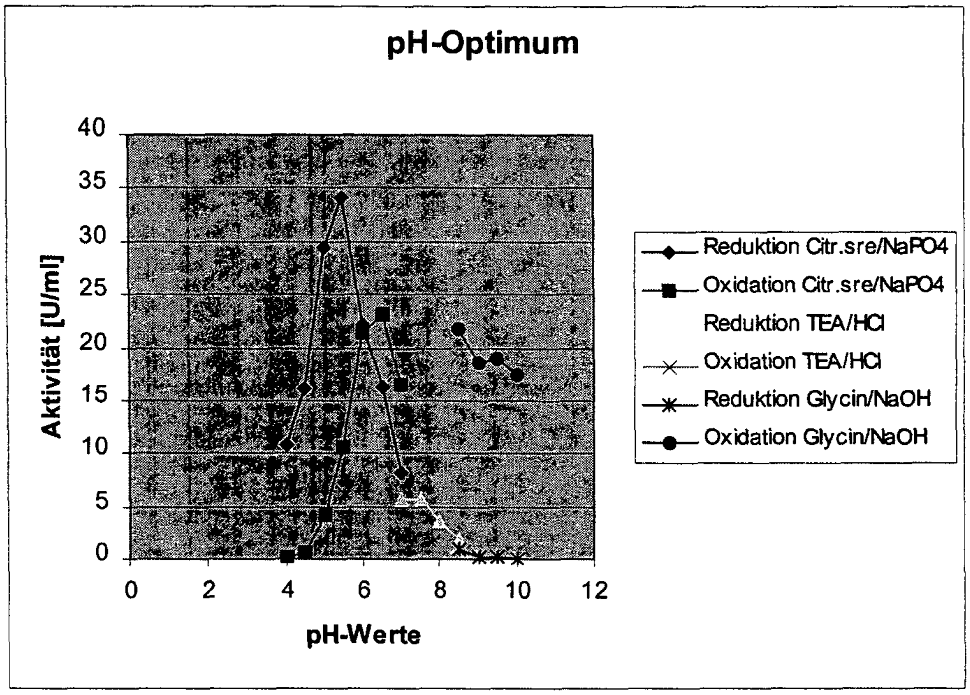
pH-Optimum
Aktivität [U/ml]
40
35
30
25
20
15
10
5
0
0
2
4
6
8
10
12
pH-Werte
Reduktion Citr.sre/NaPO4
Oxidation Citr.sre/NaPO4
Reduktion TEA/HCl
Oxidation TEA/HCl
Reduktion Glycin/NaOH
Oxidation Glycin/NaOH

Fig. 3

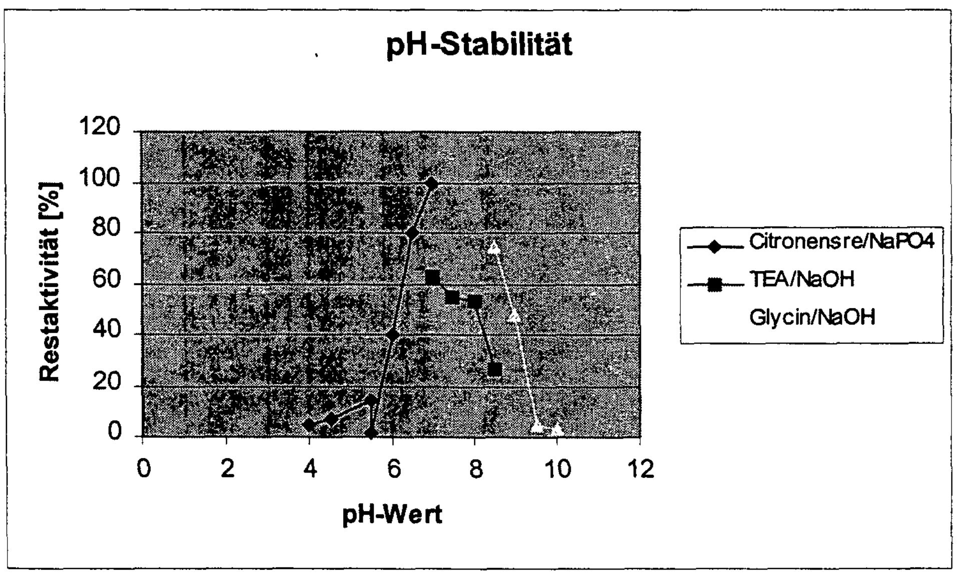
pH-Stabilität
Restaktivität [%]
120
100
80
60
40
20
0
0
2
4
6
8
10
12
pH-Wert
Citronensre/NaPO4
TEA/NaOH
Glycin/NaOH

Fig. 4

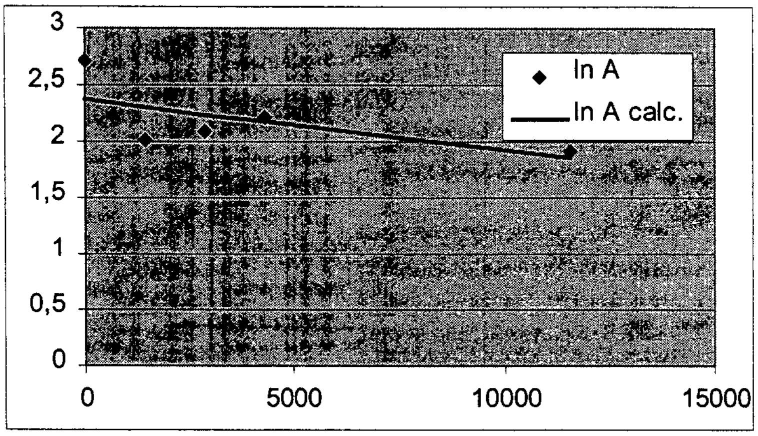
3
2,5
2
1,5
1
0,5
0
0
5000
10000
15000
ln A
ln A calc.

Fig. 5:

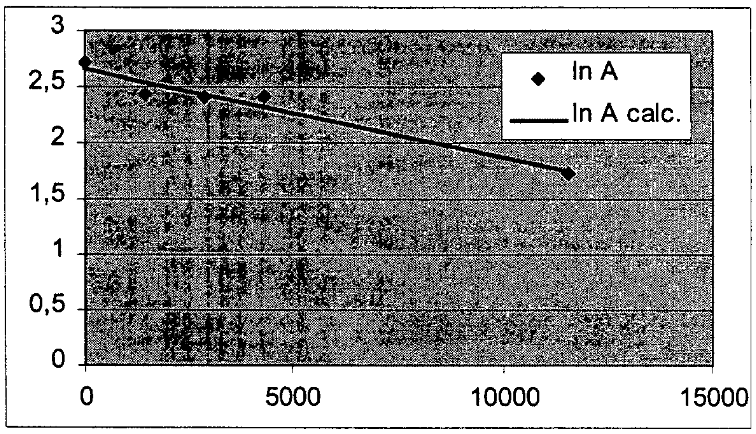
3
2,5
2
1,5
1
0,5
0
0
5000
10000
15000
ln A
ln A calc.

Fig. 6:

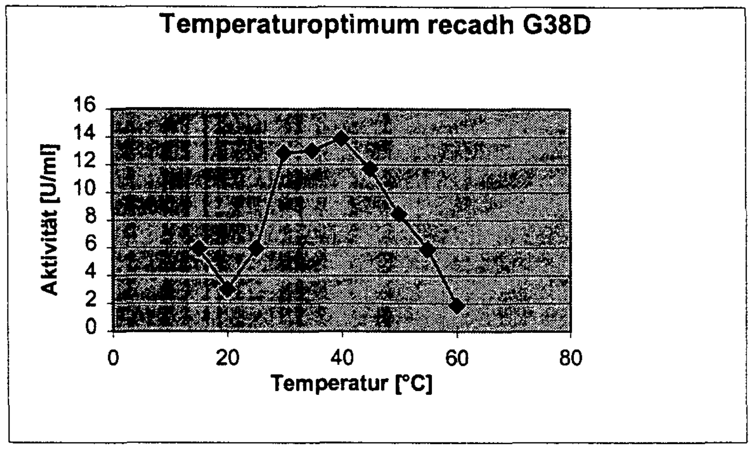


Fig.7:

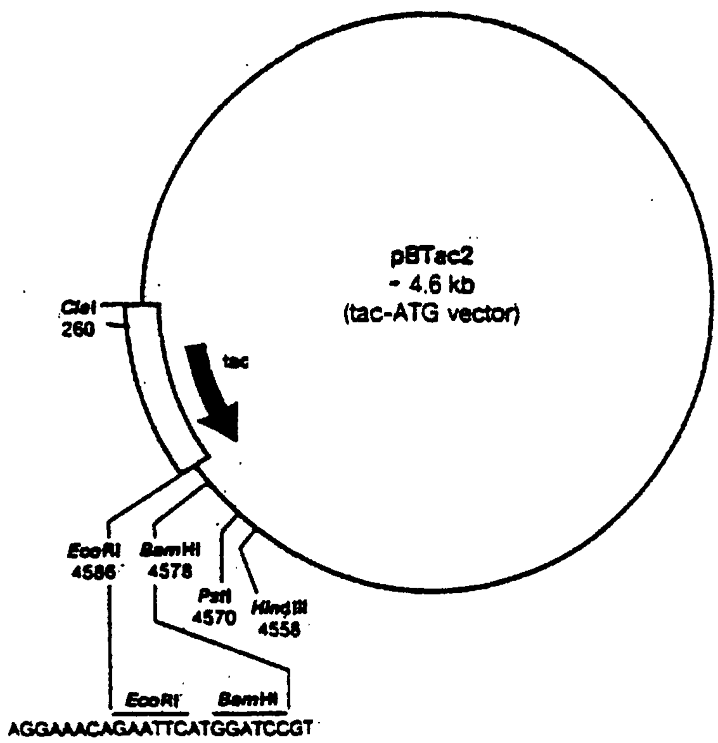


Restriction map of pBTac2 DNA:

Europäisches Patentamt

# EUROPÄISCHER TEILRECHERCHENBERICHT

der nach Regel 45 des Europäischen Patent-übereinkommens für das weitere Verfahren als europäischer Recherchenbericht gilt

Nummer der Anmeldung
EP 01 11 4953

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| A,D | WO 99 47684 A (HUMMEL WERNER ;RIEBEL BETTINA (DE); KERNFORSCHUNGSANLAGE JUELICH () 23. September 1999 (1999-09-23)<br>* Siehe Beispiel 5 (G37D, Primer) *<br>--- | 4-13 | C12N15/53<br>C12N9/04<br>C12N1/21<br>C12P7/02 |
| A,D | DE 196 10 984 A (BOEHRINGER MANNHEIM GMBH) 25. September 1997 (1997-09-25)<br>* Siehe Abbildung 1 (Sequenz) *<br>--- | 4-13 | |
| A | NAKANISHI M ET AL: "Swith of coenzyme specificity of mouse lung carbonyl reductase by substitution of threonine 38 with aspartic acid"<br>JOURNAL OF BIOLOGICAL CHEMISTRY, AMERICAN SOCIETY OF BIOLOGICAL CHEMISTS, BALTIMORE, MD, US,<br>Bd. 272, Nr. 4,<br>24. Januar 1997 (1997-01-24), Seiten 2218-2222, XP002127499<br>ISSN: 0021-9258<br>* Siehe Seite 2218 (Zusammenfassung) *<br>---<br>-/-- | 4-13 | RECHERCHIERTE SACHGEBIETE (Int.Cl.7)<br>C12N<br>C12P |

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| BERLIN | 1. November 2001 | Korsner, S-E |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

Europäisches Patentamt

**UNVOLLSTÄNDIGE RECHERCHE**
**ERGÄNZUNGSBLATT C**

Nummer der Anmeldung
EP 01 11 4953

Vollständig recherchierte Ansprüche:
4,8

Unvollständig recherchierte Ansprüche:
5-7,9-13

Nicht recherchierte Ansprüche:
1-3

Grund für die Beschränkung der Recherche:

Die geltenden Patentansprüche 1-3 und 5-7,9-13 (teilweise) beziehen sich auf Enzyme (und ihre Verwendung) charakterisiert durch eine erstrebenswerte Eigenheit oder Eigenschaft, nämlich einer durch Mutation(en) erhöhter NAD(H)-Akzeptanz.

Die Patentansprüche umfassen daher alle Produkte etc., die diese Eigenheit aufweisen, wohingegen die Patentanmeldung Stütze durch die Beschreibung im Sinne von Art. 83 EPÜ nur für eine begrenzte Zahl solcher Produkte etc. liefert - nämlich ADH (L.brevis, L.kefir) mit dem G38D Austauch.

Im vorliegenden Fall fehlen den Patentansprüchen die entsprechende Stütze bzw. der Patentanmeldung die nötige Offenbarung in einem solchen Maße, daß eine sinnvolle Recherche über den gesamten erstrebten Schutzbereich unmöglich erscheint. Desungeachtet fehlt den Patentansprüchen auch die in Art. 84 EPÜ geforderte Klarheit, nachdem in ihnen versucht wird, die Enzyme über das jeweils erstrebte Ergebnis zu definieren. Auch dieser Mangel an Klarheit ist dergestalt, dass er eine sinnvolle Recherche über den erstrebten Schutzbereich unmöglich macht. Daher wurde die Recherche auf die Teile der Patentansprüche gerichtet, welche im o.a. Sinne als klar, gestützt oder offenbart erscheinen, nämlich die Teile betreffend die Enzyme des Anspruchs 4 und der entsprechenden Unteransprüche).

Europäisches Patentamt

**EUROPÄISCHER TEILRECHERCHENBERICHT**

Nummer der Anmeldung
EP 01 11 4953

| EINSCHLÄGIGE DOKUMENTE | | | KLASSIFIKATION DER ANMELDUNG (Int.Cl.7) |
|---|---|---|---|
| Kategorie | Kennzeichnung des Dokuments mit Angabe, soweit erforderlich der maßgeblichen Teile | Betrifft Anspruch | |
| A | DATABASE 'Online!<br>1. Mai 2000 (2000-05-01)<br>LEE ET AL: "Beta-hydroxysteroid dehydrogenase"<br>retrieved from EBI/SWALL, accession no. Q9RNK5<br>Database accession no. Q9RNK5<br>XP002181542<br>* Siehe die Sequenz (.egakv..tD) *<br>----- | 4-13 | |
| | | | RECHERCHIERTE SACHGEBIETE (Int.Cl.7) |

EPO FORM 1503 03.82 (P04C12)

**ANHANG ZUM EUROPÄISCHEN RECHERCHENBERICHT ÜBER DIE EUROPÄISCHE PATENTANMELDUNG NR.** EP 01 11 4953

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-11-2001

| Im Recherchenbericht angeführtes Patentdokument | | Datum der Veröffentlichung | Mitglied(er) der Patentfamilie | | | Datum der Veröffentlichung |
|---|---|---|---|---|---|---|
| WO 9947684 | A | 23-09-1999 | DE | 19812004 | A1 | 30-09-1999 |
| | | | WO | 9947684 | A2 | 23-09-1999 |
| | | | EP | 1002097 | A2 | 24-05-2000 |
| DE 19610984 | A | 25-09-1997 | DE | 19610984 | A1 | 25-09-1997 |
| | | | EP | 0796914 | A2 | 24-09-1997 |
| | | | JP | 10028590 | A | 03-02-1998 |
| | | | US | 6225099 | B1 | 01-05-2001 |
| | | | US | 6037158 | A | 14-03-2000 |

EPO FORM P0461

Für nähere Einzelheiten zu diesem Anhang : siehe Amtsblatt des Europäischen Patentamts, Nr.12/82